# EUROPÄISCHE PATENTSCHRIFT

(11) **EP 1 764 086 B1**
(45) Veröffentlichungstag und Bekanntmachung des Hinweises auf die Patenterteilung: **29.10.2008**
(21) Anmeldenummer: 06015203.0
(22) Anmeldetag: 21.07.2006
(51) Int. Cl.: A61K 8/97, A61K 8/92, A61Q 19/00, A61Q 13/00

(54) **Mittel zur Körper- und Schönheitspflege, enthaltend wenigstens drei Baumextrakte**
Composition for body care and beauty, comprising at least three tree extracts
Composition de soin et de beauté du corps, comprenant au moins trois extraits d'arbres

(30) Priorität: 14.09.2005 DE 102005043714
(43) Veröffentlichungstag der Anmeldung: 21.03.2007
(73) Patentinhaber: Föhrdes, Friedhelm, 77770 Durbach (DE)
(72) Erfinder: Föhrdes, Friedhelm, 77770 Durbach (DE)
(74) Vertreter: Maucher, Wolfgang

(56) Entgegenhaltungen:
- WO-A-01/66079
- DE-A1- 4 238 509
- FR-A1- 2 743 722
- HU-B- 190 391
- US-B1- 6 579 543

## Beschreibung

Die Erfindung betrifft ein Mittel zur Körper- und Schönheitspflege, das Wasser, zumindest ein ätherisches Öl sowie verschiedene Baumextrakte aufweist.

Man kennt bereits zahlreiche Mittel zur Körper- und Schönheitspflege. Die vorbekannten Körper- und Schönheitspflegemittel weisen meist Bestandteile auf, die zur Pflege der Haut bestimmt sind. Dies können beispielsweise Pflegeöle sein, die mit Hilfe von Emulgatoren in Wasser gelöst sind. Zusätzlich können auch ätherische Öle und Blumen- oder Blütenextrakte enthalten sein, welche die Haut ebenfalls positiv beeinflussen und dem vorbekannten Körper- und Schönheitspflegemittel einen angenehmen Duft geben sollen.

Aus der US-B1-6 579 543, der WO 01 /66079 A, der DE 42 38 509 A1, der FR-A1-2 743 722 sowie der HU 190 391 B sind bereits verschiedene medizinisch-kosmetische und veterinärmedizinische Präparate zur äußeren Anwendung bekannt, die alle neben Wasser auch zumindest ein ätherisches Öl sowie ein Baumextrakt aufweisen. Unter anderem auch aus diesen vorbekannten Druckschriften ist die hohe Wirksamkeit ätherischer Öle in Verbindung mit bestimmten Baumextrakten bekannte, welche die Verwendung dieser Wirkstoffe für medizinisch-therapeutische Zwecke sinnvoll erscheinen lässt. Diese vorbekannten Wirkstoffe sind jedoch im wesentlichen für medizinisch-therapeutische Zwecke bestimmt, während demgegenüber ein vorrangig körper- oder schönheitspflegender Zweck mit diesen vorbekannten Wirkstoffkombinationen nicht angestrebt wird.

Um auch das Energiefeld des Anwenders positiv beeinflussen zu können, sieht die vorliegende Erfindung vor, dass das erfindungsgemäße Körper- und Schönheitspflegemittel Wasser, ätherische Ölmischungen, zumindest einen Farbstoff sowie wenigstens drei Baumextrakte aus einer Auswahl von Ulme, Buche, Roter Kastanie, Ginkgo, Linde, Eiche und Birke aufweist.

In dem erfindungsgemäßen Körper- und Schönheitspflegemittel sind neben Wasser und einer ätherischen Ölmischung auch wenigstens drei Baumextrakte aus einer Auswahl von Ulme, Buche, Roter Kastanie, Ginkgo, Linde, Eiche und Birke enthalten. Durch die Beimischung von Baumextrakten zu Wasser sowie einer ätherischen Ölmischung zeichnet sich das erfindungegemäße Körper- und Schönheitspflegemittel durch eine positive Beeinflussung und eine harmonische Balance des Energiefelds des Anwenders aus. Auch der im erfindungsgemäßen Körper- und Schönheitspflegemittel enthaltene, zumindest eine Farbstoff vermag das Energiefeld des Anwenders vorteilhaft zu beeinflussen.

Diese positiven Wirkungen auf das Energiefeld des Anwenders werden noch zusätzlich begünstigt, wenn zumindest eine Teilmenge des Wassers Quellwasser ist.

Vorteilhaft ist es, wenn das Mittel zumindest ein Blumen- oder Blütenextrakt enthält.

Soweit das erfindungsgemäße Mittel auch Blumen- beziehungsweise Blütenextrakte enthält, sehen bevorzugte Ausführungsformen vor, dass das Mittel Blumen- beziehungsweise Blütenextrakte von Arnika, Ringelblume, Johanniskraut, Wegwarte, Rose, Jasmin und/oder Lavendel enthält.

Weitere bevorzugte Ausführungsformen sehen vor, dass das Mit - tel ätherische Öle von Palmarosa, Lavendel, Rose, Blauer Kamilde und/oder Veilchen enthält.

Zusätzlich oder stattdessen kann es vorteilhaft sein, wenn das Mittel ätherische Ölmischungen oder -kompositionen von Zitrone, Zitronenmelisse und Grapefruit und/oder von Orange, Mandarine und Sandelholz enthält,

Um die hautpflegende Wirkung des erfindungsgemäßen Körper- und Schönheitspflegemittels zu begünstigen, kann es vorteilhaft sein, wenn das Mittel weitere Hautpflegemittel enthält.

Bevorzugte Weiterbildungen gemäß der Erfindung sehen vor, dass das Mittel als Bodylotion, Duschcreme oder Gesichtscreme ausgebildet ist.

## Patentansprüche

1. Mittel zur Körper- und Schönheitspflege, das Wasser, ätherische Ölmischungen, zumindest einen Farbstoff sowie wenigstens drei Baumextrakte aus einer Auswahl von Ulme, Buche, Roter Kastanie, Ginkgo, Linde, Eiche und Birke aufweist.

2. Mittel nach Anspruch 1, **dadurch gekennzeichnet, dass** zumindest eine Teilmenge des Wassers Quellwasser ist.

3. Mittel nach Anspruch 1 oder 2, **dadurch gekennzeichnet, dass** das Mittel zumindest ein Blumen- oder Blütenextrakt enthält.

4. Mittel nach einem der Ansprüche 1 bis 3, **dadurch gekennzeichnet, dass** das Mittel Blumenbeziehungsweise Blütenextrakte von Arnika, Ringelblume, Johanniskraut, Wegwarte, Rose, Jasmin und/oder Lavendel enthält.

5. Mittel nach einem der Ansprüche 1 bis 4, **dadurch gekennzeichnet, dass** das Mittel ätherische Öle von Palmarosa, Lavendel, Rose, Blauer Kamille und/oder Veilchen enthält.

6. Mittel nach einem der Ansprüche 1 bis 5, **dadurch gekennzeichnet, dass** das Mittel ätherische Ölmischungen von Zitrone, Zitronenmelisse und Grapefruit und/oder von Orange, Mandarine und Sandelholz enthält.

7. Mittel nach einem der Ansprüche 1 bis 6, **dadurch gekennzeichnet, dass** das Mittel weitere Hautpflegemittel, enthält .

8. Mittel nach einem der Ansprüche 1 bis 7, **dadurch gekennzeichnet, dass** das Mittel als Bodylotion, Duschcreme oder Gesichtscreme ausgebildet ist.

## Claims

1. Agent for body and beauty care which comprises water, mixtures of ethereal oils, at least one colouring agent and at least three tree extracts selected from among elm, beech, red chestnut, ginkgo, lime, oak and birch.

2. Agent according to claim 1, **characterised in that** at least some of the water is spring water.

3. Agent according to claim 1 or 2, **characterised in that** the agent contains at least one extract of flowers or blossom.

4. Agent according to one of claims 1 to 3, **characterised in that** the agent contains flower or blossom extracts from arnica, marigold, St. John's wort, chicory, rose, jasmine and/or lavender.

5. Agent according to one of claims 1 to 4, **characterised in that** the agent contains ethereal oils from palmarosa, lavender, rose, blue camomile and/or violet.

6. Agent according to one of claims 1 to 5, **characterised in that** the agent contains mixtures of ethereal oils from lemon, lemon balm and grapefruit and/or from orange, mandarin and sandalwood.

7. Agent according to one of claims 1 to 6, **characterised in that** the agent contains further skin care agents.

8. Agent according to one of claims 1 to 7, **characterised in that** the agent is in the form of a body lotion, shower cream or face cream.

## Revendications

1. Agent de soins corporels et de soins de beauté qui comprend de l'eau, des mélanges d'huiles essentielles, au moins un colorant, ainsi qu'au moins trois extraits d'arbres parmi la sélection des arbres suivants : orme, hêtre, marronnier rouge, ginkgo, tilleul, chêne et bouleau.

2. Agent selon la revendication 1, **caractérisé en ce qu'**au moins une fraction de l'eau est de l'eau de source.

3. Agent selon la revendication 1 ou 2, **caractérisé en ce que** l'agent contient au moins un extrait de fleur.

4. Agent selon l'une des revendications 1 à 3, **caractérisé en ce que** l'agent contient des extraits de fleur d'arnica, de souci, de millepertuis, de plantain, de rose, de jasmin et/ou de lavande.

5. Agent selon l'une des revendications 1 à 4, **caractérisé en ce que** l'agent contient des huiles essentielles de palmarosa, de lavande, de rose, de camomille bleue et/ou de violette.

6. Agent selon l'une des revendications 1 à 5, **caractérisé en ce que** l'agent contient des mélanges d'huiles essentielles de citron, de mélisse officinale et de pamplemousse et/ou d'orange, de mandarine et de santal.

7. Agent selon l'une des revendications 1 à 6, **caractérisé en ce que** l'agent contient d'autres agents de soin de la peau.

8. Agent selon l'une des revendications 1 à 7, **caractérisé en ce que** l'agent est élaboré sous la forme d'une lotion corporelle, d'une crème pour la douche ou d'une crème pour le visage.
